(19) Europäisches Patentamt
European Patent Office
Office européen des brevets

(11) Veröffentlichungsnummer : **0 277 527 B1**

(12) **EUROPÄISCHE PATENTSCHRIFT**

(45) Veröffentlichungstag der Patentschrift :
**27.03.91 Patentblatt 91/13**

(51) Int. Cl.$^5$ : **A61F 2/24**

(21) Anmeldenummer : **88100556.5**

(22) Anmeldetag : **16.01.88**

(54) **Herzklappenprothese.**

(30) Priorität : **22.01.87 DE 3701704**

(43) Veröffentlichungstag der Anmeldung :
**10.08.88 Patentblatt 88/32**

(45) Bekanntmachung des Hinweises auf die Patenterteilung :
**27.03.91 Patentblatt 91/13**

(84) Benannte Vertragsstaaten :
**AT BE CH DE ES FR GB GR IT LI LU NL SE**

(56) Entgegenhaltungen :
**EP-A- 0 070 259**
**EP-A- 0 133 608**
**WO-A-85/04094**
**DE-A- 2 753 159**
**ENGINEERING IN MEDICINE, Band 16, Nr. 2, April 1987, Seiten 67-76, MEP Ltd, London, GB; H. REUL et al.: "Laboratory testing of prosthetic heart valves"**

(73) Patentinhaber : **B. Braun Melsungen AG**
**Carl-Braun Strasse**
**W-3508 Melsungen (DE)**

(72) Erfinder : **Knoch, Martin, Dr. Ing.**
**Kullenhofwinkel 34**
**W-5100 Aachen (DE)**
Erfinder : **Reul, Helmut, Prof. Dr.-Ing.**
**Akazienstrasse 65**
**W-5160 Düren (DE)**
Erfinder : **Rau, Günter, Prof. Dr. rer. nat.**
**Fuchserde 50**
**W-5100 Aachen (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

EP 0 277 527 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Herzklappenprothese nach dem Oberbegriff des Patentanspruchs 1.

Die natürlichen Herzklappen sind drei- bzw. zweiflügelige Segelklappen, die technisch gesprochen die Funktion von Rückschlagventilen haben, welche das Blut in einer Richtung durchlassen, in Gegenrichtung jedoch sperren. Beim Ersatz der natürlichen Herzklappen durch mechanische Pendel- oder Kippscheibenprothesen werden einflügelige oder zweiflügelige Klappen eingesetzt, bei denen klappenförmige Schließkörper in einem Klappenring, der an der betreffenden Öffnung des Herzens festgenäht wird, durch den Blutdruck bzw. die Blutströmung bewegbar sind. Beim Langzeiteinsatz solcher Herzklappenprothesen können sich schwerwiegende Probleme ergeben, die eine lebenslange Einnahme von Antikoagulantien durch den Patienten oder das Auswechseln der Prothese erforderlich machen.

Maßgeblich für das strömungstechnische Verhalten des Schließkörpers einer Herzklappenprothese ist dessen Skelettlinie. Als Skelettlinie bezeichnet man die quer zur Schwenkachse verlaufende Mittellinie des Schließkörperquerschnitts. Bekannt sind (EP-A-0 133 608) zweiflügelige Herzklappenprothesen mit halbkreisförmigen oder halbelliptischen Schließkörpern mit gerader oder kreisbogenförmig gewölbter Skelettlinie. Ferner sind zweiflügelige Herzklappenprothesen bekannt (WO 85/04094), bei denen die Schließkörper quer zur Skelettlinie gewölbt sind, um den Querschnitt der durch die beiden Schließkörper begrenzten mittleren Öffnung zu vergrößern, ohne den engsten Querschnitt des sich dadurch ergebenden Kanals ebenfalls vergrößern zu müssen. Die Wölbung quer zur Skelettlinie dient ausschließlich der Vergrößerung des mittleren der drei Teilquerschnitte, sowie der Vergrößerung der Öffnungs- und Schließmomente. In geöffneter Stellung sind die Schließkörper aus der Ringachse heraus zum Ringumfang hin gewölbt.

Bei den bekannten zweiflügeligen Herzklappenprothesen mit planen Schließkörpern beträgt der maximale Öffnungswinkel, bei dem der Schließkörper gegen einen Anschlag stößt, etwa 85° zur Ringaustrittsebene, damit die Klappe im Öffnungszustand durch die Strömung fest gegen den Anschlag gedrückt wird, und damit beim Rückströmen ein sicheres Schließen des Schließkörpers gewährleistet ist. Die Schließkörper bilden demnach in der Hauptströmungsphase in Strömungsrichtung gesehen einen diffusorähnlichen Querschnittsverlauf des mittleren Durchlasses. An den scharfkantigen Anströmkanten wird die Strömung seitlich abgelenkt, wodurch an diesen Anströmkanten im mittleren Teilquerschnitt eine Strömungsablösung erfolgt. Die Strömung legt sich anschließend wahllos wieder an einen der Schließkörper an. Dadurch entsteht hinter dem anderen Schließkörper ein ausgedehnter Strömungsnachlauf mit stark fluktuierender Wirbelbildung. Dieser Zustand kann bei jedem Schlagzyklus wechseln. Das Öffnungsverhalten der beiden Schließkörper ist aufgrund der unterschiedlichen Druckverteilungen ungleichmäßig. Ein ähnliches regelloses Strömungsverhalten ergibt sich auch bei Schließkörpern mit kreisbogenförmig gewölbter Skelettlinie. Bei solchen Schließkörpern, die sich, ausgehend von der engsten Stelle des zwischen ihnen gebildeten Querschnitts, diffusorähnlich erweitern, reißt die Strömung im Bereich der Querschnittserweiterung ab.

Strömungsablösungen und Totwassergebiete, die sich hinter der Herzklappenprothese im Bereich der Aorta ascendens ergeben, sind bei allen bisher bekannten Schließkörperausführungen festzustellen. Infolge der auftretenden hohen Schubspannungen können sich Blutschädigungen ergeben ; durch die stark wechselnden Strömungsverhältnisse entstehen starke örtliche Belastungen der Aortenwand. Nachteilig ist außerdem, daß sich im Bereich der Aortenbulben Totwassergebiete bilden, aus denen das Blut unzureichend abströmt, so daß eine erhöhte Gefahr der Trombenbildung entsteht.

Der Erfindung liegt die Aufgabe zugrunde, eine Herzklappenprothese der im Oberbegriff des Patentanspruchs 1 angegebenen Art zu schaffen, die eine weitgehend gleichmäßige und zentrale Durchströmung der Aortenbulben und der Aorta ascendens sicherstellt und bei der an den Schließkörpern im geöffneten Zustand keine Strömungsablösungen auftreten, wobei keine wesentliche Ablenkung der Blutströmung verursacht wird.

Die Lösung dieser Aufgabe erfolgt erfindungsgemäß mit den im kennzeichnenden Teil des Patentanspruchs 1 angegebenen Merkmalen.

Bei der erfindungsgemäßen Herzklappenprothese sind die Schließkörper so geformt, daß sie in der Öffnungsstellung im Mittelschnitt eine Venturidüse bilden, deren hinter der engsten Stelle liegender Diffusorbereich sich im Querschnitt linear erweitert. Diese Querschnittserweiterung ist so gering, daß keine Strömungsablösung erfolgen kann. Andererseits werden die Schließkörper durch die von außen gegen die divergierenden Bereiche drückende Strömung und durch den Sog, der sich an der hinter den Schwenkachsen liegenden engsten Stelle des Düsenquerschnitts ergibt, sicher in der Öffnungsstellung gehalten und gegen ihre Anschläge gedrückt. Die Düsenwände der sich in der Öffnungsstellung der Schließkörper ergebenden Düse sind nur im konvergierenden Einlaufbereich gekrümmt, während sie im divergierenden Auslaufbereich geradlinig ausgebildet sind. Die engste Stelle des Düsenquerschnitts befindet sich in Strömungsrichtung gesehen hinter den Schwenkachsen. Eintrittsbreite und Austrittsbreite der Düse sind im wesentlichen einander gleich. Der minimal mögliche Krümmungsradius der Skelettlinie auf der Anströmseite wird dadurch begrenzt, daß in

geöffneter Stellung keine Strömungsablösungen an den Schließkörperoberflächen auftreten dürfen. Zu diesem Zweck können die Schließkörper im Anströmbereich auch mit variabler Dicke ausgeführt werden. Die Skelettlinienkontur stromab von der Stelle des engsten Querschnitts (in der Öffnungsstellung der Schließkörper) ist vorzugsweise geradlinig und beschreibt einen Diffusoröffnungswinkel zwischen 3° und 5° zur Ringachse, so daß auch hier keine Ablösungen auftreten.

Im Gegensatz zu Schließkörpern mit geraden Skelettlinien kann der Öffnungswinkel zwischen der die Schließkörperenden verbindenden Sehne und der Ringaustrittsebene 90° betragen. Das erforderliche Drehmoment zum Schließen wird bei Druckumkehr durch den Aufstau im rückwärts durchströmten Venturiquerschnitt der Düse bewirkt. Beim Öffnen wird der mit zunehmendem Öffnungswinkel nachlassende Überdruck auf den konkaven Schließkörperaußenseiten durch den an der Stelle engsten Querschnitts entstehenden Unterdruck unterstützt. Die Schließkörper werden dadurch zuverlässig gegen die konstruktiv vorgegebenen Anschläge gezogen.

Die erfindungsgemäße Herzklappenprothese bietet in Verbindung mit einem düsenförmigen Klappenring, dessen Öffnungsquerschnitt sich in Strömungsrichtung stetig (d.h. knickfrei) verringert, die Möglichkeit einer völlig ablösungsfreien Klappendurchströmung. Die Strömung wird im Gegensatz zur einflügeligen Bauform nicht aus der achsparallelen Richtung abgelenkt und liegt aufgrund der sehr geringen Strömungsbeeinflussung bereits kurz hinter den Aortenbulben am gesamten Umfang der Aorta ascendens an. Der Druckverlust liegt deutlich unter dem bisher niedrigsten Druckverlust bekannter mechanischer Klappenprothesen. Es ist zu erwarten, daß die postoperative Behandlung mit Antikoagulantien weitgehend eingeschränkt werden kann.

Ein wesentlicher Vorteil der in den Ansprüchen 6 und 7 angegebenen zusätzlichen Wölbung quer zur Skelettlinie besteht darin, daß die Schwenkachsen unter Einhaltung des festgelegten engsten Querschnitts zwischen den geöffneten Schließkörpern größere Abstände zueinander haben. Dadurch wächst der wandnahe Strömungsimpuls im Bereich der Schließkörperlagerung und die Gefahr einer Trombenansammlung wird verringert.

Vorteilhafte Weiterbildungen und Ausgestaltungen der Erfindung sind in den Unteransprüchen 2 bis 7 angegeben.

Im folgenden werden unter Bezugnahme auf die Zeichnungen Ausführungsbeispiele der Erfindung näher erläutert.

Es zeigen :

Fig. 1 einen Querschnitt durch eine implantierte Herzklappenprothese,

Fig. 2 einen Schnitt entlang der Linie II-II von Fig. 1,

Fig. 3 eine Ansicht eines Schließkörpers,

Fig. 4 einen Schnitt des Schließkörpers entlang der Linie IV-IV von Fig. 3 zur Erläuterung der Schließkörpergeometrie,

Fig. 5 ein zweites Ausführungsbeispiel der Herzklappenprothese im implantierten Zustand,

Fig. 6 einen Schnitt entlang der Linie VI-VI von Fig. 5,

Fig. 7 in ähnlicher Darstellung wie Fig. 2 eine Ansicht einer Herzklappenprothese, deren Schließkörper 12 im geöffneten Zustand einen Venturikanal mit parallelen Wandteilen bilden und

Fig. 8 eine modifizierte Querschnittsform eines Schließkörpers in ähnlicher Darstellung wie Fig. 4.

Die Herzklappenprothese nach den Fign. 1 bis 4 weist einen kreisförmigen Klappenring 10 auf, dessen Innenfläche 10a sich in Strömungsrichtung stetig zunehmend verengt. Der Klappenring 10 ist von einem Nahtring 11 umgeben, der an das Aortengewebe 15 angenäht werden kann. Im Klappenring 10 sind die beiden Schließkörper 12 schwenkbar gelagert. Die Schwenkachsen 13, die sich quer durch den Ring 10, etwa in der Mittelebene des Ringes erstrecken, sind exzentrisch angeordnet und haben jeweils den gleichen Abstand von der Ringachse 14. Beide Schwenkachsen 13 verlaufen parallel zueinander. Es handelt sich nicht um physische Achsen sondern um geometrische Achsen, auf denen an jedem Schließkörper zeigen Lagerzapfen angeordnet sind, die nach entgegengesetzten Seiten von dem Rand des Schließkörpers abstehen und in entsprechende (nicht dargestellte) Lagerausnehmungen des Klappenrings 10 eintauchen.

Jeder Schließkörper 12 besteht aus einem starren Flächenmaterial, dessen Wandstärke vorzugsweise an allen Stellen gleich ist. Jeder Schließkörper 12 hat eine im wesentlichen halbkreisförmige oder halbelliptische Form (Fig. 3), wobei die abgeschrägte bogenförmige Kante 16 sich im Schließzustand gegen die Innenfläche 10a des Klappenringes 10 legt, während die geradlinigen Kanten 17 beider Schließkörper in der Mitte des Klappenrings 10 stumpfwinkelig gegeneinanderstoßen und eine Dichtfläche bilden. Jeder Klappenflügel 12 ist (im Schließzustand) in Strömungsrichtung ausgebaucht. In der Öffnungsstellung bilden die Klappenflügel 12 eine Venturidüse, deren Strömungsquerschnitt sich in Strömungsrichtung zunächst verringert und dann anschließend linear zunimmt. Die engste Stelle befindet sich hinter den Schwenkachsen 13. Der gegenseitige Abstand der Schließkörper am Einlaß der Venturidüse ist gleich dem gegenseitigen Abstand am Auslaß.

In Fig. 4 ist der Verlauf der Skelettlinie 19 eines Schließkörpers 12 dargestellt. Mit Skelettlinie ist diejenige

Mittellinie des Profils bezeichnet, die in einer quer zur Schwenkachse 13 verlaufenden Ebene liegt, und zwar in der Quermittelebene des Schließkörpers. In dieser Ebene ist die Struktur des Schließkörpers am stärksten ausgeprägt; mit zunehmendem Abstand von dieser Mittelebene nach außen wird der Schließkörper immer flacher, wobei sich die der Skelettlinie 19 entsprechende Mittellinie immer mehr der Sehnenebene annähert.

Der im Bereich der Lagerachse 13 liegende Abschnitt 19a der Skelettlinie 19 ist bogenförmig ausgebildet. Er hat einen kreisbogenförmigen Verlauf mit dem Radius r um eine parallel zur Schwenkachse 13 verlaufende Achse. Die Schwenkachse 13 befindet sich etwa in der Mitte des bogenförmigen Abschnitts 19a. An den bogenförmigen Abschnitt 19a schließt sich tangential der gerade Abschnitt 19b an, dessen Länge annähernd gleich derjenigen des bogenförmigen Abschnitts ist.

Die Länge der Sehne 20 zwischen den Kanten 17 und 16 ist mit a bezeichnet und der Abstand der Schwenkachse 13 von der Kante 17 ist mit e bezeichnet. Der Quotient e : a beträgt vorzugsweise 0,15 bis 0,25. Der Winkel $\alpha_{s1}$ zwischen der Tangente 21 an das Ende des bogenförmigen Skelettlinien-Abschnitts 19a an der Kante 17 und der Sehne 20 beträgt zwischen 5° und 20°, vorzugsweise zwischen 10° und 15°. Der Winkel $\alpha_{s2}$ zwischen dem geraden Abschnitt 19b und der Sehne 20 beträgt vorzugsweise 3° bis 5°. Der Radius r des bogenförmigen Abschnitts 19a beträgt

$$r = \frac{\sin \alpha_{s2}}{1 - \cos(\alpha_{s1} + \alpha_{s2})} \cdot a .$$

Das durch den Mittelpunkt M des Kreises des bogenförmigen Abschnitts 19a hindurchgehende Lot L auf die Sehne 20 schneidet die Sehne 20 im Abstand k von der Kante 17. Der Abstand des Punktes P, in dem die Abschnitte 19a und 19b gegeneinanderstoßen, von der Kante 17 beträgt t. Für die Größen k und t gelten die folgenden Beziehungen:

$$k = r \sin \alpha_{s1} > e$$
$$t = r (\sin \alpha_{s1} + \sin \alpha_{s2}).$$

In Fig. 1 sind die Schließkörper 12 in Öffnungsstellung und in Schließstellung abgebildet, wobei die sich in der Öffnungsstellung ergebenden Stromlinien angegeben sind. Die Herzklappenprothese ist am Eingang der Aorta ascendens 22 angeordnet. Es handelt sich bei dem vorliegenden Ausführungsbeispiel also um eine Aortenklappe. Die Prothese ist in gleicher Weise auch bei entsprechender Modifikation des textilen Nahtrings 11 als Mitralklappe verwendbar. Hinter der Herzklappe befinden sich in der Aorta ascendens drei sternförmig angeordnete Aortenbulben 24, die die Segel der natürlichen dreiflügeligen Herzklappe im Öffnungszustand umgeben. Wenn die natürliche Herzklappe entfernt und durch die Herzklappenprothese ersetzt ist, beeinflussen die Aortenbulben 24 die Blutströmung, wobei Rezirkulationsgebiete bzw. Totwasserbereiche entstehen können. Wie in den Fign. 1 und 2 dargestellt ist, wird die Herzklappenprothese so eingesetzt, daß die Schließkörper 12 im Öffnungszustand etwa parallel zu dem einen Aortenbulbus 24 verlaufen. Wie erkennbar ist, weisen die Enden 16 und 17 in Richtung der abgehenden bzw. entgegen der ankommenden Strömung. Die durch die Enden 16 und 17 in der Skelettlinie 19 hindurchgehenden Sehnen 20 verlaufen unter einem Winkel von 90° zur Ringebene, so daß Eintrittsquerschnitt und Austrittsquerschnitt der zwischen den Schließkörpern 12 gebildeten Düse einander gleich sind. Die Strömung wird durch die Schließkörper 12 nicht wesentlich abgelenkt oder regellos verwirbelt.

Bei geöffneten Schließkörpern 12 bezeichnet B den Abstand zwischen den Kanten 17 der Schließkörper am Düseneinlaß. Der Abstand der Kanten 16 am Düsenauslaß beträgt ebenfalls B. Der gegenseitige Abstand der Schließkörper 12 an der engsten Stelle der Düse, also im Abstand k vom Einlaßende, beträgt b. Es gelten die folgenden Richtwerte:

$$\frac{b}{a} = 0{,}20 \text{ bis } 0{,}35$$
$$\frac{b}{B} = 0{,}65 \text{ bis } 0{,}85.$$

Bei der in den Fign. 5 und 6 dargestellten Herzklappenprothese ist der Klappenring 10 in gleicher Weise ausgebildet wie bei dem ersten Ausführungsbeispiel und auch die Skelettlinie der Schließkörper 26 entspricht derjenigen der Schließkörper des ersten Ausführungsbeispiels. Der Unterschied besteht darin, daß jeder Schließkörper 26 zusätzlich quer zur Ebene der Skelettlinie, die der Schnittebene von Fig. 5 entspricht, gewölbt

ist. Die Schließkörper sind in geöffneter Stellung von den Drehachsen 13 zur Ringachse 14 hin gewölbt. Durch diese Wölbung wird erreicht, daß die Schwenkachsen 13 bei festgelegtem minimalem Abstand der Schließkörper zueinander weiter auseinanderrücken und einen größeren Abstand zur zugehörigen Skelettlinie erhalten. Bei Beibehaltung des Düsenquerschnitts in der Skelettlinienebene (Schnittebene von Fig. 5) wird der Düsenquerschnitt insgesamt vergrößert, so daß der Querschnitt der mittleren Öffnung den Querschnitten der beiden äußeren Öffnungen weiter angenähert ist.

Ferner werden für das Auswaschverhalten ungünstige spitze Winkel an den Lagerstellen zwischen Ringinnenfläche und den Schließkörperoberflächen vermieden.

Bei der Herzklappenprothese nach Fig. 7 haben die Schließkörper 12 im Bereich der Skelettlinienebene die gleiche Querschnittsform wie der Schließkörper nach Fig. 4. Quer zur Skelettlinienebene bildet jeder Schließkörper 12 einen geradlinigen Mittelabschnitt 12a, an den sich die in geöffneter Stellung zur Drehachse 13 nach außen gewölbten Endabschnitte 12b anschließen. Jeder Endabschnitt 12b verläuft bei geöffneten Schließkörpern vorzugsweise unter einem rechten Winkel zu der Innenfläche des Klappenrings 10, gegen den er stößt. Im Öffnungszustand verlaufen die Mittelabschnitte 12a parallel zueinander, während die Endabschnitte 12b der beiden Schließkörper 12 divergieren. Zwischen den Schließkörpern 12 wird ein Venturikanal mit parallelen Wandteilen gebildet. Diese Ausführungsform vereint die Vorteile der Herzklappen nach den Fign. 1, 2 und 5, 6 und gibt einen größeren Gestaltungsspielraum zur Abstimmung des günstigsten Abstandes e der Schwenkachse 13 von der Kante 17 der Fign. 3 und 4, unabhängig vom gewählten Düsenquerschnitt in der Skelettlinienebene.

Fig. 8 zeigt eine andere Ausführungsform des Profils des Schließkörpers in der Skelettlinienebene. Während der Schließkörper nach Fig. 4 aus Material konstanter Wandstärke besteht, weisen der Schließkörper nach Fig. 8 im Bereich des Skelettlinienabschnitts 19a eine variierende Dickenverteilung auf. Diese variierende Dickenverteilung dient zur Vermeidung von Strömungsablösungen an der konkaven Schließkörperoberfläche, insbesondere für den Fall, daß $\alpha_{s1}$ größer als 15° ist.

## Ansprüche

1. Herzklappenprothese mit einem Klappenring (10), in dem zwei Schließkörper (12 ; 26) um jeweils eine quer und mit Abstand zur Ringachse (14) verlaufende Schwenkachse (13) schwenkbar gelagert sind, wobei jede Schwentachse (13) ihren zugehörigen Schließkörper in einen küzeren und einen längeren Flügelabschnitt unterteilt und im Schließzustand die kürzeren Flügelabschnitte der Schließkörper (12 ; 26) gegeneinanderliegen, während die längeren Flügelabschnitte am Klappenring (10) anliegen und die Schließkörper im aufgeklappten Zustand einen mittleren Durchlaß des Klappenringes (10) begrenzen, **dadurch gekennzeichnet**, daß die in einer Normalenebene zur Schwenkachse (13) verlaufende Skelettlinie (19) des Querschnitts eines jeden Schließkörpers (12 ; 26) aus einem bogenförmigen Abschnitt (19a), der sich etwa über die halbe Länge des Schließkörpers erstreckt, und einem geraden Abschnitt (19b), der sich über die restliche Länge des Schließkörpers erstreckt, besteht, wobei der bogenförmige Abschnitt (19a) im Bereich der Schwenkachse (13) angeordnet und seine konkave seite in geschlossenem zustand des Schließkörpers stromaufwärts gerichtet ist.

2. Herzklappenprothese nach Anspruch 1, dadurch gekennzeichnet, daß die Tangente (21) an das Ende des bogenförmigen Abschnitts (19a) mit einer durch die Enden (16,17) der Skelettlinie (19) hindurchgehenden Sehne (20) einen Winkel ($\alpha_{s1}$) von 10° bis 15° bildet.

3. Herzklappenprothese nach Anspruch 1 oder 2, dadurch gekennzeichnet, daß der gerade Abschnitt (19b) mit einer durch die Enden (16,17) der Skelettlinie (19) hindurchgehenden Sehne (20) einen Winkel ($\alpha_{s2}$) von 3° bis 5° bildet.

4. Herzklappenprothese nach einem der Ansprüche 1 bis 3, dadurch gekennzeichnet, daß die Schwenkachse (13) im Bereich der Mitte des bogenförmigen Abschnitts (19a) angeordnet ist.

5. Herzklappenprothese nach einem der Ansprüche 1 bis 4, dadurch gekennzeichnet, daß im aufgeklappten Zustand die durch die Enden (16,17) der Skelettlinie (19) hindurchgehenden Sehnen (20) parallel zur Ringachse (14) verlaufen.

6. Herzklappenprothese nach einem der Ansprüche 1 bis 5, dadurch gekennzeichnet, daß jeder Schließkörper (26) zusätzlich quer zu der die Skelettlinie (19) enthaltenden Normalenebene zur Schwenkachse (13) in geöffneter Stellung aus der Schwenkachse (13) heraus zur Ringachse (14) hin gewölbt ist, wobei die beidseitig der Skelettlinie (19) angeordneten Hälften symmetrisch zueinander sind.

7. Herzklappenprothese nach Anspruch 6, dadurch gekennzeichnet, daß jeder Schließkörper (12) einen geraden Mittelabschnitt (12a) und daran anschließende, in geöffneter Stellung zur Drehachse 13 nach außen gewölbte Endabschnitte (12b) aufweist, und daß die Mittelabschnitte (12a) der beiden Schließkörper (12) einen Venturikanal mit parallelen Seitenwandteilen bilden.

## Claims

1. Cardiac valve prosthesis comprising a valve ring (10) in which two closing bodies (12 ; 26) are supported to be pivotal about a respective swivel axis (13) extending transversely and spaced from the ring axis (14), each swivel axis (13) dividing their associated closing body into a shorter and a longer wing section and the shorter wing sections of the closing bodies (12 ; 26) resting against each other in the closing position, while the longer wing sections adjoin the valve ring (10) and the closing bodies, in tilted open condition, confining a central passage of the valve ring (10), **characterized in that** the skeleton line (19) of the cross section of each closing body (12 ; 26) extending in a normal plane to the swivel axis (13) consists of an arcuate portion (19a) extending substantially over half the length of the closing body, and of a straight portion (19b) extending over the residual length of the closing body, the arcuate portion (19a) being provided near the swivel axis (13), and that its concave side is directed upstream when the closing body is in the closed state.

2. Cardiac valve prosthesis as defined in claim 1, characterized in that the tangent (21) to the end of the arcuate portion (19a) forms with a chord (20) traversing the ends (16,17) of the skeleton line (19) an angle ($\alpha_{s1}$) of 10° to 15°.

3. Cardiac valve prosthesis as defined in claim 1 or 2, characterized in that the straight portion (19b) forms with a chord (20) traversing the ends (16,17) of the skeleton line (19) an angle ($\alpha_{s2}$) of 3° to 5°.

4. Cardiac valve prosthesis as defined in one of claims 1 to 3, characterized in that the swivel axis (13) is provided in the central region of the arcuate portion (19a).

5. Cardiac valve prosthesis as defined in one of claims 1 to 4, characterized in that, in open condition, the cords (20) through the ends (16, 17) of the skeleton line (19) extend in parallel to the ring axis (14).

6. Cardiac valve prosthesis as defined in one of claims 1 to 5, characterized in that each closing body (26) is curved additionally transversely to the normal plane to the swivel axis (13) towards the ring axis (14), the halves separated by the skeleton line (19) being symmetrical with respect to each other.

7. Cardiac valve prosthesis as defined in claim 6, characterized in that each closing body (12) comprises a straight central portion (12a) and, adjoined thereto, end sections (12b) outwardly curved in opened position to the swivel axis (13), and that the central sections (12a) of both closing bodies (12) form a Venturi passage having parallel side wall portions.

## Revendications

1. Prothèse de valve cardiaque comportant un anneau de valve (10), dans lequel sont logés à pivotement deux corps de fermeture (12 ; 26) tournant chacun autour d'un axe de pivotement (13) de tracé perpendiculaire à l'axe (14) de l'anneau et distant de celui-ci, chaque axe de pivotement (13) divisant le corps de fermeture qui lui est associé en une partie d'ailes plus courtes et une partie d'ailes plus longues et les parties d'ailes plus courtes des corps de fermeture (12 ; 26) s'appuyant l'une sur l'autre dans l'état de fermeture, alors que les parties d'ailes plus longues s'appuient sur l'anneau de valve (10), et que les corps de fermeture limitent, dans l'état ouvert, un passage médian de l'anneau de valve (10), **caractérisé en ce que** la ligne d'ossature (19) de la section transversale de chaque corps de fermeture (12 ; 26), ligne dont le tracé est dans un plan normal à l'axe de pivotement (13), se compose d'une partie (19a) en forme d'arc qui s'étend à peu près sur la demi-longueur du corps de fermeture et d'une partie droite (19b) qui s'étend sur le reste de la longueur du corps de fermeture, la partie en forme d'arc (19a) étant disposée dans la zone de l'axe de pivotement (13) et son côté concave étant orienté vers l'amont du corps de fermeture dans l'état fermé.

2. Prothèse de valve cardiaque selon la revendication 1, caractérisée en ce que la tangente (21) à l'extrémité de la partie en forme d'arc (19a) forme, avec une corde (20) passant par les extrémités (16, 17) de la ligne d'ossature (19), un angle ($\alpha_{s1}$) de 10° à 15°.

3. Prothèse de valve cardiaque selon la revendication 1 ou 2, caractérisée en ce que la partie droite (19b) forme, avec une corde (20) passant par les extrémités (16, 17) de la ligne d'ossature (19), un angle ($\alpha_{s2}$) de 3° à 5°.

4. Prothèse de valve cardiaque selon l'une des revendications 1 à 3, caractérisée en ce que l'axe de pivotement (13) est disposé dans la zone médiane de la partie en forme d'arc (19a).

5. Prothèse de valve cardiaque selon l'une des revendications 1 à 4, caractérisée en ce que, dans l'état ouvert, les cordes (20) passant par les extrémités (16, 17) de la ligne d'ossature (19) sont parallèles à l'axe (14) de l'anneau.

6. Prothèse de valve cardiaque selon l'une des revendications 1 à 5, caractérisée en ce que chaque corps de fermeture (26) est en outre incurvé, transversalement au plan normal de l'axe de pivotement (13) contenant la ligne d'ossature (19), vers l'extérieur à partir de l'axe (14) de l'anneau dans la position ouverte, les moitiés

disposées des deux côtes de la ligne d'ossature (19) étant symétriques l'une de l'autre.

7. Prothèse de valve cardiaque selon la revendication 6, caractérisée en ce que chaque corps de fermeture (12) présente une partie médiane droite (12a) et des parties d'extrémité (12b), incurvées vers l'extérieur par rapport à l'axe de rotation (13) dans la position ouverte, se raccordant à la partie médiane, et en ce que les parties médianes (12a) des deux corps de fermeture (12) constituent un canal de Venturi comportant des parties de parois latérales parallèles.

FIG.1

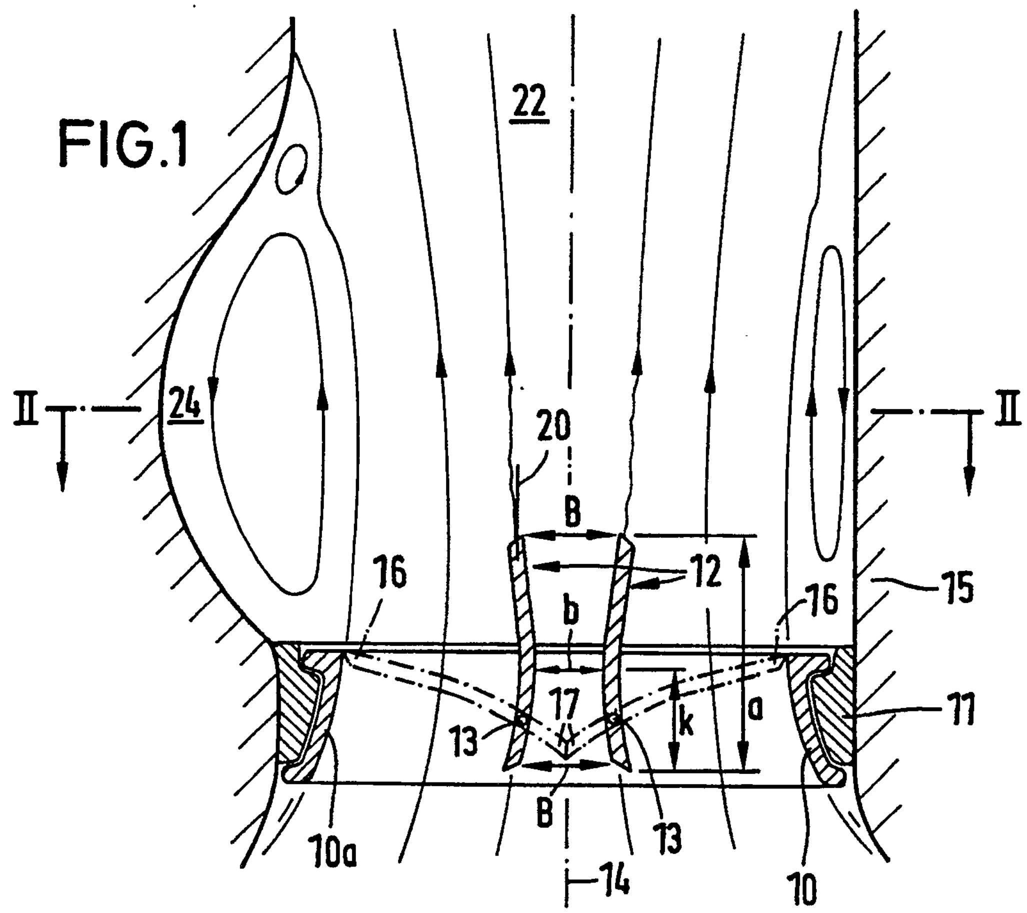
22
II
24
II
20
B
12
16
b
16
15
17
k
a
11
13
13
B
13
10a
14
10


FIG.2

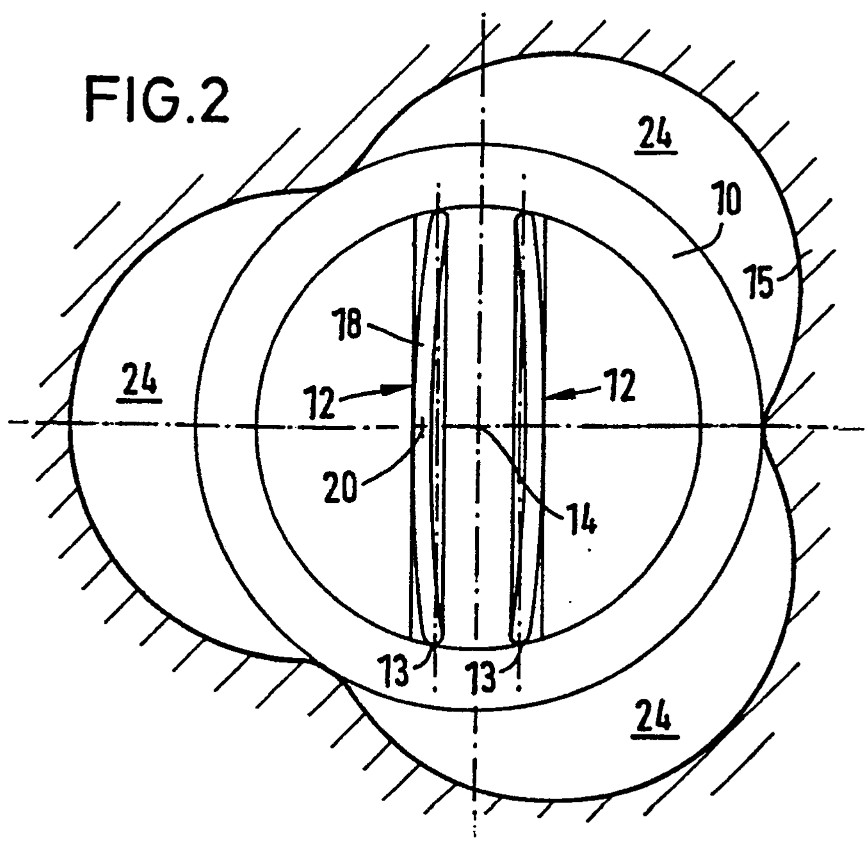
24
10
15
18
24
12
12
20
14
13
13
24

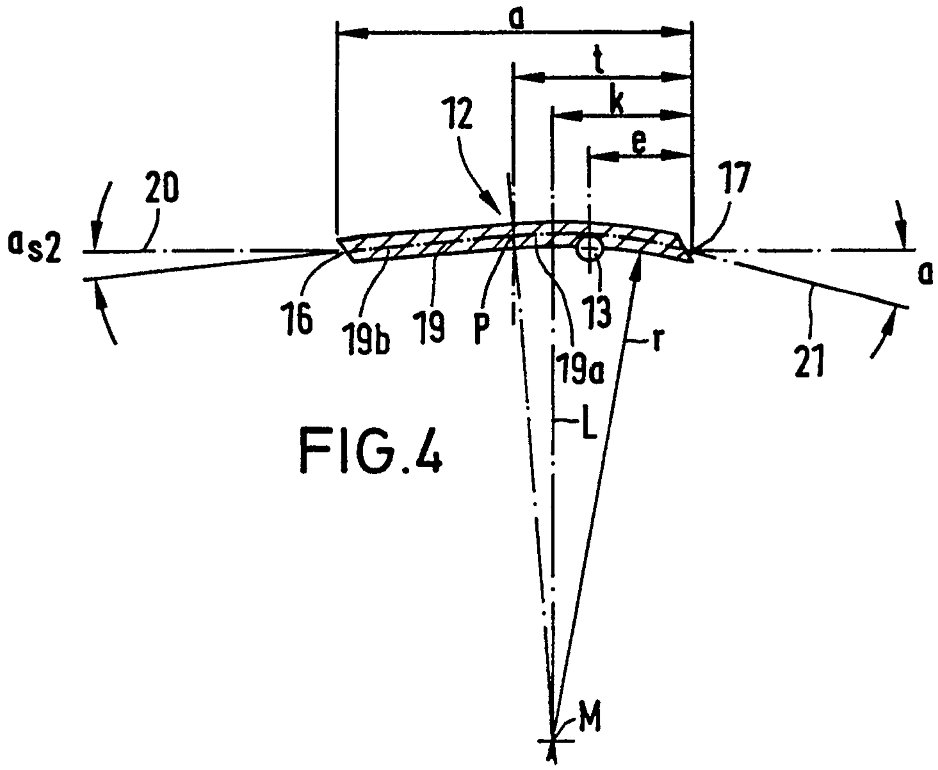


FIG.4

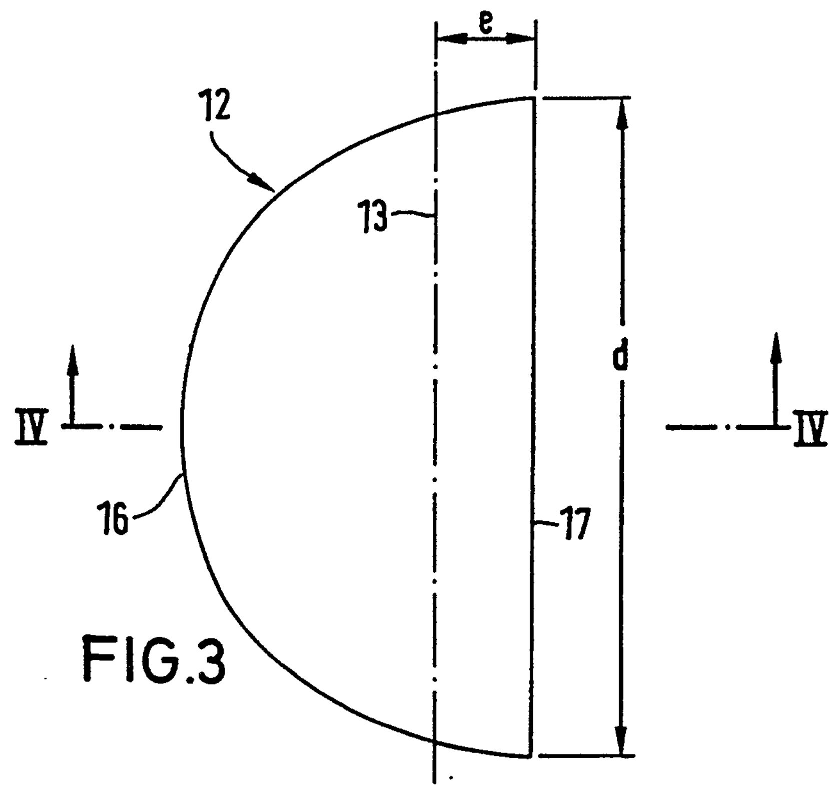


FIG.3

FIG.5

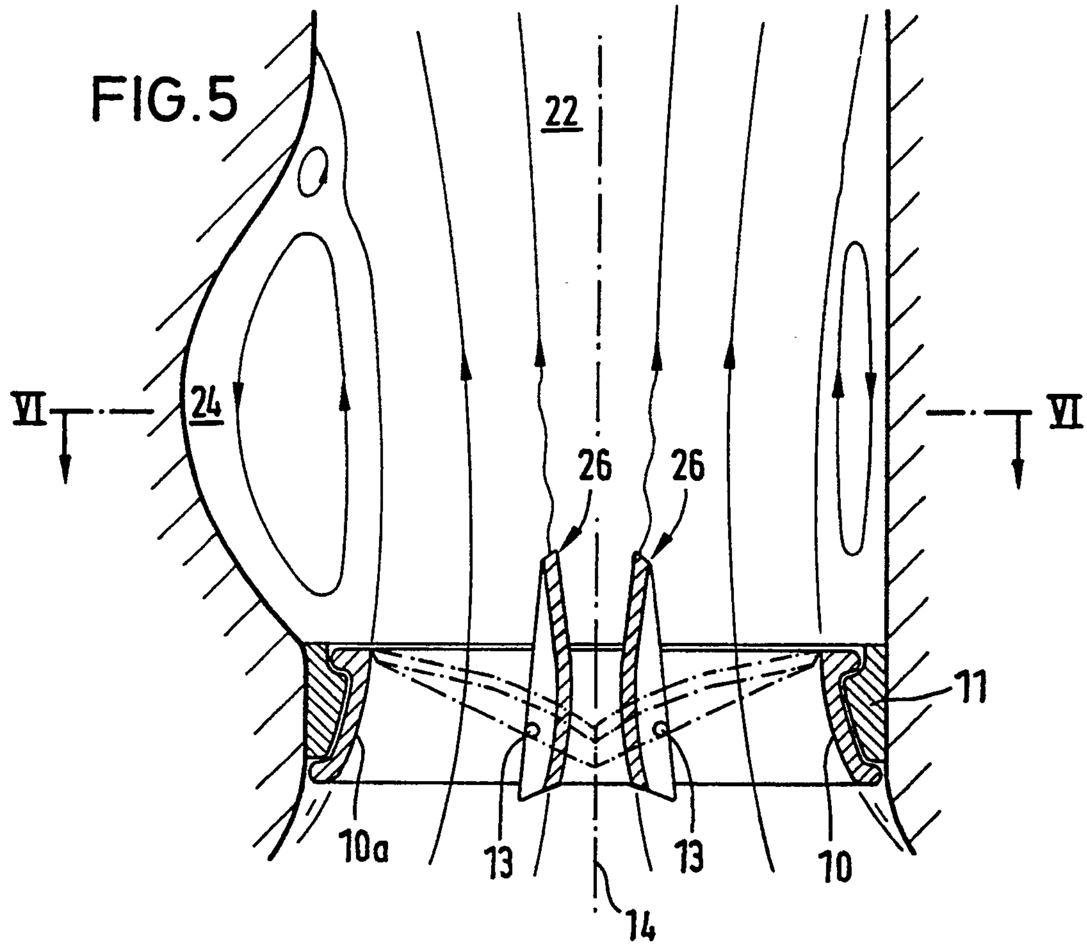


FIG.6

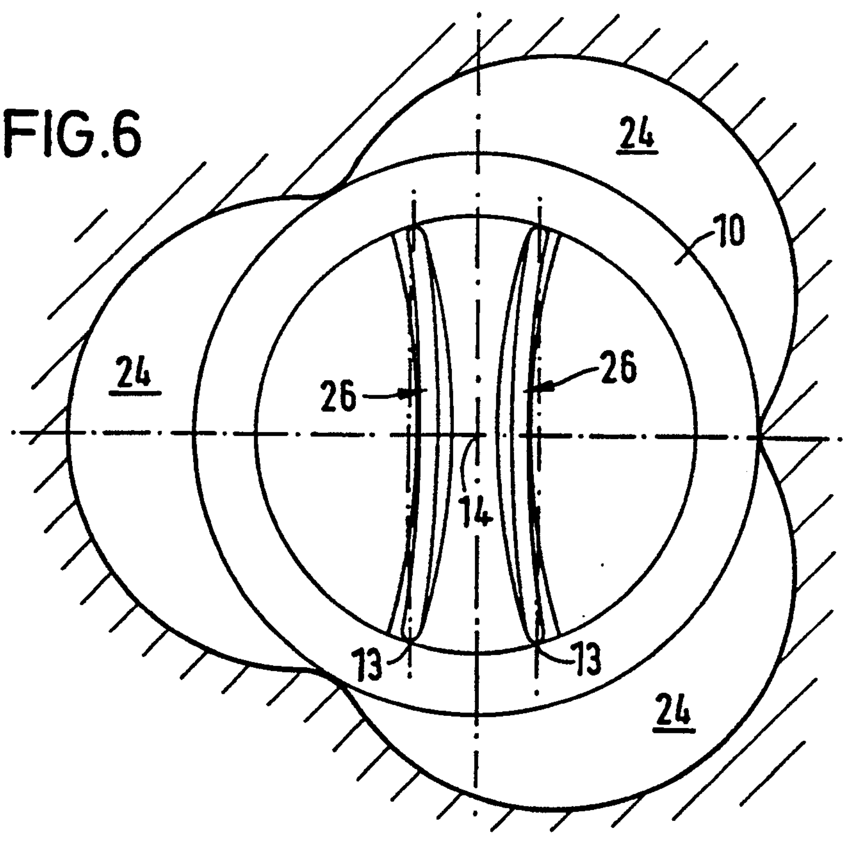

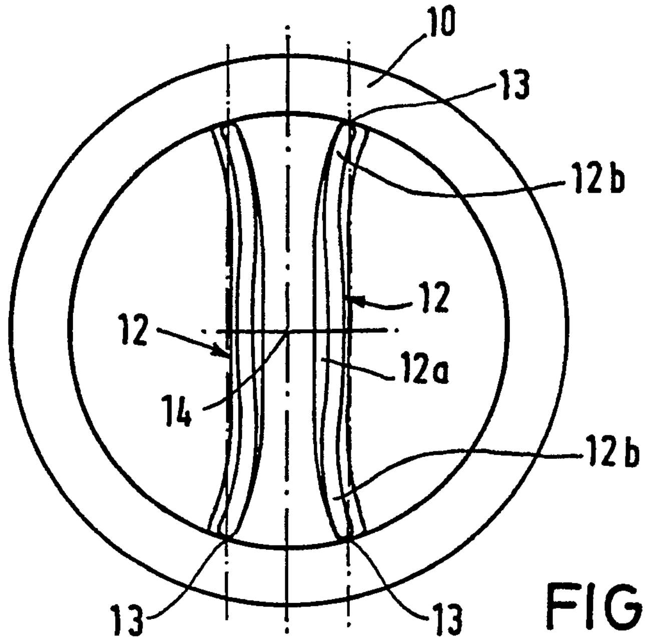


FIG.7

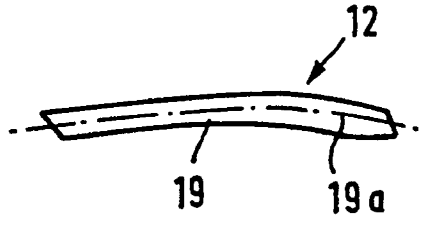


FIG.8